# EUROPEAN PATENT APPLICATION

(11) **EP 1 106 181 A1**
(43) Date of publication of application: **13.06.2001**
(21) Application number: 99937017.4
(22) Date of filing: 11.08.1999
(51) Int. Cl.: A61K 31/66, A61K 31/195, A61K 38/01, A61K 38/16, A61K 38/40, A61K 35/78, A61K 31/23, A61K 31/20, A61K 31/70, A61K 33/00

(54) **NUTRITIONAL COMPOSITIONS FOR PREVENTING OR TREATING HYPERLIPOPROTEINEMIA**

(30) Priority: 12.08.1998 JP 22834298
(71) Applicant: MORINAGA MILK INDUSTRY CO., LTD., Minato-ku, Tokyo-to 108-0014 (JP)
(72) Inventor: INOUE, Nariaki, Tokyo 134-0088 (JP); HAYASAWA, Hirotoshi, 5-1-83, Higashira Zama Kanagawa 228-0004 (JP); TAKASE, Mitsunori, 5-1-83, Higashira Zama Kanagawa 228-0004 (JP); SHIMIZU, Takashi, 5-1-83, Higashira Zama Kanagawa 228-0004 (JP); OSHIDA, Kyoichi, 5-1-83, Higashira Zama Kanagawa 228-0004 (JP)
(74) Representative: Pett, Christopher Phineas
(86) International application number: JP9904336
(87) International publication number: WO0009138

(57) **Abstract**

The invention provides a nutrient composition for the prevention or treatment of hyperlipoproteinemia, wherein the nutrient composition contains one or two or more bitrogen sources selected from the group consisting of protein, protein hydrolysates, peptides and amino acid, lipids, carbohydrate, vitamins and minerals, wherein the lipid comprises one of the following a) or b):
a) phospholipids of at least 5.0 % (by weight) of the total solid content;
b) phospholipids of at least 5.0 % (by weight) of the total solid content, and fats and oils of less than 7.0 % (by weight) of the total solid content (provided that 0 % (by weight) is excluded). This nutrient composition does not cause essential fatty acid deficiency, can supply sufficient energy without increasing the level of undesirable fat in blood and is, therefore, useful for the prevention or treatment of Type I or Type V hyperlipoproteinemia.

## Description

### Technical Field

The present invention relates to a nutrient composition effective for the prevention or therapeutic treatment of hyper lipoproteinemia such as the deficiency of lipoprotein lipase (abbreviated as LPL hereinafter), hyperchylomicronemia (also known as Type I hyperlipoproteinemia or Burger Grutz syndrome) and Type V hyperlipoproteinemia (hyperlipemia).

### Background of the Invention

Recently, diseases involving the elevation of lipid level in blood have increased. The following are such diseases known so far.

In hyperchylomicronemia, chylomicron is observed in blood plasma in the early morning while in hunger, and emulsification of the blood plasma occurs. The deficiency of LPL or the decrease in its activity, and the deficiency of apo C-II, one of the proteins composing a lipoprotein particle, are said to be its cause.

Meanwhile, hyperlipemia has conventionally referred to symptoms of turbidity in serum caused by the increase of triglyceride concentration. However, currently, hyperlipemia is used as a synonym for hyperlipoproteinemia. This is because the pathology of hyperlipemia is associated with the increase of one or more lipid component of serum, which are cholesterol, triglyceride and phospholipid, while serum lipid components exist in the form of lipoproteins, in which lipid components are bound to proteins (apoproteins) in blood.

The pathology of hyperlipoproteinemia involves the increase of serum lipoprotein. Serum lipoproteins are classified into chylomicrons, very low density lipoproteins (referred to as VLDL hereinafter), low density lipoproteins (referred to as LDL hereinafter) and high density lipoproteins (referred to as HDL hereinafter), and for other abnormal lipoproteins, β-VLDL.

Hyperlipoproteinemia is classified into six phenomenological types according to the lipoprotein that increases (written in parenthesis in the following description); Type I (chylomicron), Type IIa (LDL), Type IIb (LDL and VLDL), Type III (β-VLDL), Type IV (VLDL) and Type V (chylomicron and VLDL). There are secondary diseases arising from primary diseases and essential diseases based on constitution (heredity) , which are accompanied by symptoms such as xanthoma, arteriosclerosis, abdominal pain and pancreatitis, depending on its phenomenological type. (The above descriptions are based on Shigeru Goto et al. ed., Newest Medical Dictionary (Saishin Igaku Dai-jiten), second edition, pages 521, 533 and 566, Ishiyaku Shuppan, 1996). Furthermore, the various types of the disease mentioned above are all referred to as hyperlipoproteinemia in the following description, except for those descriptions cited from references of the conventional technology.

Conventionally, a low fat nutrition method which reduces the triglyceride concentration in chylomicron has been applied as alimentary therapy for patients with Type I and Type V hyperlipoproteinemia. Because the intake of exogenous lipid is reduced by this low fat nutrition method, the formation of chylomicron in blood is reduced, resulting in the decrease of serum triglyceride. However, because prominent increase of serum triglyceride level is observed when a patient ingests food containing edible fats and oils comprising triglycerides with n-6 and n-3 fatty acids for the purpose of obtaining essential fatty acid supply, generally, limitation of the intake of such long chain triglycerides has been chosen for alimentary therapy.

Thus, in the conventional alimentary therapy, to which patients with Type I and Type V hyperlipoproteinemia were subjected, one of the nutritious roles of lipid, the supply of essential fatty acids to bio-organisms, was not sufficiently displayed. Furthermore, it was known that the reduction of the composition of essential fatty acids in serum is observed in patients on low-fatty acid diet for several weeks or more.

Further, in recent years, numerous nutrient compositions which are effective for the prevention or treatment of various diseases have been developed. Some conventional techniques wherein the lipid comprising the nutrient composition is characteristic are described bellow.
a) A composition prepared by blending organic phosphate compounds with eicosapolyenoic acids such as arachidonic acid, eicosapentaenoic acid, and docosahexaenoic acid (Japanese Patent Provisional Publication No. 59-219396/84).
b) A composition for the reduction or suppression of cholesterol increase, wherein 3.5 % or more of the total mass of egg yolk lecithin is contained in a normal composition comprising fat and oil (Japanese Patent Provisional Publication No. 60-97916/85).
c) A composition for reducing the levels of blood cholesterol and triglyceride, which comprises a first component selected from the group consisting of eicosapentaenoic acid, docosahexaenoic acid and a combination thereof, and a second component selected from a group consisting of di-γ-homolinolenic acid, cis-linolenic acid, γ-linolenic acid and a combination thereof, wherein the first component and the second component are blended in a ratio of 3:1 to 1:3 (Japanese Patent Provisional Publication No. 60-115522/85).
d) A composition effective for arteriosclerotic diseases and thrombotic diseases, which is an emulsion containing at least one eicosapolyenoic acid compound selected from eicosapolyenoic acid and its derivatives (Japanese Patent Provisional Publication No. 60-123414/85).
e) An anti-arteriosclerotic agent containing α-linolenic acid ester as the effective component (Japanese Patent Provisional Publication No. 60-222318/85).
f) A lipogenous infusion containing γ-linolenic acid ester as the effective component, which is effective for the prevention or treatment of arteriosclerosis, myocardial infarction, cerebral thrombosis, aorta aneurysm, peripheral artery occlusion, hyperlipemia, diabetes mellitus and so on (Japanese Patent Provisional Publication No. 60-222419/85).
g) A nutrient composition effective on the psychosomatic bioactivity of the cardiovascular system, the nervous system and the digestive system, which comprises 20 to 80 wt% of purified pine seed oil containing 5,9,12-cis-octadecatrienoic acid, 10 to 75 wt% of an oil containing one or more substance selected from γ-linolenic acid, eicosapentaenoic acid and docosahexaenoic acid, and 0.01 to 50 wt% of other oil-soluble bioactive components (Japanese Patent Provisional Publication No. 61-58536/86).
h) A tube feeding composition in the form of an O/W emulsion, which comprises sugar, protein or its decomposition product, lipid, minerals and vitamins, and contains highly unsaturated fatty acids as the lipid component, which shows effects on improving the pathology by the pharmacological action of the highly unsaturated fatty acids (Japanese Patent Provisional Publication No. 61-58560/86 and Japanese Patent Publication No. 7-48991/95).
i) A composition for the prevention and treatment of vascular, arteriosclerotic and thrombotic diseases, containing lecithin and at least one fat and oil of the following substances: eicosapentaenoic acid, docosaenoic acid and their esters (Japanese Patent Provisional Publication No. 61-112020/86).
j) A nutrient effective for the prevention or treatment of vascular disorders, which contains five or more of the seven active lipids, palmitooleic acid, γ-linolenic acid, eicosapentaenoic acid, docosahexaenoic acid, lecithin, vitamin E and octacosanol (Japanese Patent Provisional Publication No. 62-224258/87).
k) A method for adjusting serum cholesterol concentration in mammalian animals, comprising local administration of at least one phospholipid in an amount sufficient to adjust the serum cholesterol concentration (Japanese Patent Provisional Publication No. 6-5501245/94).
l) A nutritious composition comprising sugar, protein, its decomposition product or amino acid, lipid, mineral and vitamin, wherein sugar, protein, and its decomposition products or amino acid comprise the abundant component, and the lipid contains fats and oils containing 2 to 40 wt% of poly-unsaturated fatty acid in the fatty acid composition, as well as 0.1 to 30 parts by weight per 100 parts by weight of the oil-soluble components of lecithin containing at least 10 wt% of phosphatidylcholine with 60 wt% or more acetone-insoluble substances and 5 wt% or more of phosphatidylethanolamine, which shows actions to improve serum lipid level and to suppress platelet aggregation (Japanese Patent No. 2537645).

Additionally, reviews concerning extraction and fractionation of lipid and other natural products using liquefied gas and super-critical liquid have been published (Oil Chemistry, Vol. 31, No. 7, pp. 411-413, 1982), while a method for recovering high-purity lecithin from which neutral fat are removed, comprising treating animal and vegetable lecithin containing various phospholipids and neutral.fats with solvents, or a method for fractionating a specific phospholipid wherein lecithin is purified by solvent-removal and drying using liquefied gas (for example, liquefied carbon dioxide and so on) (Japanese Patent Provisional Publication No. 60-94987/85).

As apparent from the above description of the conventional techniques, various nutrient compositions for the purpose of improving blood lipid level by utilizing the bioactivity of poly-unsaturated fatty acids and/or phospholipids such as lecithin have been known, including for example, a composition which is effective in decreasing or inhibiting the increase of blood cholesterol by using egg yolk lecithin (Japanese Patent Provisional Publication No. 60-97916/85). However, these compositions are intended for decreasing or inhibiting the increase of cholesterol and/or triglyceride in endogenous lipoprotein generated in vivo by utilizing poly-unsaturated fatty acid and/or phospholipid, and could not be expected to show the same effects on Type I and Type V hyperlipoproteinemia, pathologies caused by increased chylomicron due to exogenous, namely diet-induced intake of long chain fatty acid triglyceride.

For the prevention or treatment of Type I and Type V hyperlipoproteinemia, it is effective to strictly control the intake of exogenous (namely, diet-induced) long chain fatty acid triglyceride, as described above. However, a patient subjected to a diet in which long chain fatty acid triglyceride, a supply source of essential fatty acid, is restricted may fall into a state of essential fatty acid-deficiency in a few weeks.

Based on these reasons, no nutrient composition effective for the prevention or treatment of Type I and Type V hyperlipoproteinemia has been known, and such effective nutrient composition has long been desired.

Under the circumstances described above, the purpose of the present invention is to provide a nutrient composition effective for the prevention or treatment of Type I and Type V hyperlipoproteinemia.

### Disclosure of the Invention

As a result of thorough investigations of various nutrient compositions effective for the treatment Type I and Type V hyperlipoproteinemia, the inventors of the present invention found that even though essential fatty acids of the triglyceride-type exist in blood chylomicron of such patients after the intake of fats and oils, the ratio of 5,8,11-eicosatrienoic acid, an n-9 series fatty acid in the plasma phospholipid increases significantly compared to that of a healthy person, resulting from the decrease in the amount of n-6 and n-3 series essential fatty acids in the tissue, due to the hindrance of fatty acid incorporation into tissue.

Additionally, by measuring the phospholipase activity in patients with Type I and Type V hyperlipoproteinemia, the inventors have found that in some patients, the level of activity was almost equal to that of a healthy person, and that the intake of phospholipid by such patients result in the release of the essential fatty acids which are bound to the phospholipid incorporated in blood lipoprotein through the action of phospholipase, which are then incorporated into tissues, causing an increase in the composition of essential fatty acids in plasma phospholipid, while decreasing that of eicosatrienoic acid, thereby improving the condition of essential fatty acid deficiency.

Based on these novel findings, the inventors have scientifically verified that the intake of phospholipid or phospholipid with a specific amount of fats and oils is effective as an alimentary therapy for patients with Type I and Type V hyperlipoproteinemia, thus completing the present invention of the nutrient composition.

Therefore, the role of phospholipid in the present invention is to promote the specific intake of essential fatty acids to tissues in patients with Type I and Type V hyper lipoproteinemia, and completely differs from conventional technology in terms of its physiological action.

In other words, as a means to solve the above problems, the present invention provides a nutrient composition for the prevention or treatment of hyperlipoproteinemia, wherein the nutrient composition contains one or two or more nitrogen sources selected from the group consisting of protein, protein hydrolysates, peptides and amino acid, lipids, carbohydrate, vitamins and minerals, wherein the lipid comprises one of the following a) or b):
a) phospholipids of at least 5.0 % (by weight) of the total solid content;
b) phospholipids of at least 5.0 % (by weight) of the total solid content, and fats and oils of less than 7.0 % (by weight) of the total solid content (provided that 0 % (by weight) is excluded).

Also, a preferable embodiment of the nutrient composition of the present invention is that the content of fats and oils occupies 0.1 to 2.0 % (by weight) of the total solid content and that the purity of the phospholipid is at least 80 % (by weight) (the same is true for the following unless otherwise stated).

### Best Mode for Carrying Out the Invention

The lipid used in the nutrient composition of the present invention comprises one of the following:
a) at least 5.0 %, preferably 7.0 to 15.0 % of phospholipid in the total solid content; or
b) at least 5.0 %, preferably 7.0 to 15.0 % of phospholipid in the total solid content and less than 7.0 %, preferably 0.1 to 2.0 of fats and oils in the total solid content.

Additionally, in either of the cases, the purity of phospholipid is preferably 80 % or more.

Useful fats and oils include, for example, soy bean oil and safflower oil which are generally used as edible-oil, and the so-called lauric acid-series fats and oils such as natural coconut oil and palm nucleic oil. Other medium chain triglycerides (MCT) produced by the ester reaction of medium chain fatty acid and glycerin may also be used.

When a patient shows LPL activity, a composition containing fats and oils in the aforementioned amounts may be administered, but when patients show extremely less LPL activity, a composition containing phospholipid alone or a composition containing phospholipid and medium chain triglyceride should be administered.

Because the digestion and absorption mechanism of medium chain triglycerides differ from those of long chain triglycerides (New England Journal of Medicine, Vol. 280, p. 1045, 1969), medium chain triglyceride may be used in the nutrient composition administered to patients with no LPL activity.

As apparent from the following test examples, less than 7.0 %, preferably 0.1 to 2.0 % of the total solid content of a mixture of one or two or more of these fats and oils should be blended with the phospholipids described below. Further, the amount of these fats and oils blended may appropriately be adjusted, according to the age, symptom and so on of the patient to whom the nutrient composition is administered.

Although an example of the phospholipid used in the nutrient composition of the present invention would include soy bean phospholipid, egg yolk phospholipid, marine phospholipid derived from fish oil, phospholipid derived from rape seed oil and so on, egg yolk lecithin or soy bean lecithin which are produced in quantity in an industrial scale is preferable. For egg yolk lecithin, there are egg yolk oils containing about 30 % phospholipid, and products containing 70 % and 100 % phospholipid; products with as much phospholipid content as possible are preferable.

Furthermore, soy bean lecithin is commercially available as paste-like products (phospholipid content 62 to 68 %) produced by decolorizing the separated hydrates produced during the gum removal process of soy bean oil purification, powdery products with phospholipid contents increased to 95 % or more, and so on, and like egg yolk lecithin, products with as much phospholipid content as possible are preferable.

As apparent from the following test examples, at least 5.0%, preferably 7.0 to 15.0 % of the total solid content of these phospholipids should be blended. Further, the amount of phospholipid blended may be appropriately adjusted, according to the age, symptoms and so on of the patient to whom the nutrient composition is administered.

The nitrogen source used in the nutrient composition of the present invention comprises one or two or more substances selected from the group consisting of edible proteins derived from various animals and vegetables, their hydrolysates, peptides and amino acids. Specifically, the nitrogen source is as follows.

Edible proteins derived from various animals and vegetables include, for example, commercially available casein, whey protein, soy bean protein, egg protein and so on. Examples of the amino acids are acidic amino acids, neutral amino acids, and basic amino acids, such as aliphatic amino acids, sulfur-containing amino acids, aromatic amino acids, and oxyamino acids.

The protein hydrolysates are products of acid-hydrolysis or enzymatic hydrolysis of the various proteins mentioned above. Hydrolysates produced according to the method described in Japanese Patent No. 1,003,417 is one example, preferable for its lack of bitterness and antigenicity. According to Example 1 of the patented invention, such hydrolysates may be produced by the following method.

To 1 kg of commercially available casein (manufactured by Merck & Co., Ltd.), 9 kg of water is added to disperse the casein thoroughly, after which the pH of the mixture is adjusted to pH 7.0 by adding a 2N aqueous solution of sodium hydroxide, thereby completely dissolving the casein to obtain a casein solution of about 10 %, which is then sterilized at 85 °C for 15 minutes and cooled to 50 °C. To the resulting casein solution,1,000 activity units per 1 g protein each of ground powder of Lactobacillus helveticus (20,000 activity units/g), Pharmacopoeia pancreatin (manufactured by Amano Pharmaceutical Co., Ltd.; 25,000 activity units/g) and Amano A (manufactured by Amano Pharmaceutical Co., Ltd.; 80,000 activity units/g), are added individually, after which the resulting mixture is retained at 50 °C for 24 hours to hydrolyze the casein, then heated at 85 °C for 15 minutes to inactivate the enzyme. The mixture is then cooled, to obtain about 9.5 liters of a solution of decomposed casein.

Additionally, the peptides include, for example, fractions of the protein hydrolysates fractionated according to their molecular weight. For example, such peptides may be produced by the following method described in Japanese Patent Provisional Publication No. 59-76022/84.

50 g of dried egg white (protein content 82 %) is dissolved in 1 kg of water, after which hydrochloric acid is added to adjust the pH of the resulting mixture to pH 1. Then, 1 g each of Morcine (manufactured by Fujisawa Pharmaceutical Co., Ltd.) and Sunprose (manufactured by Hankyu Kyoei Bussan Co., Ltd.) are subsequently added. The mixture is then hydrolyzed at 40 °C for 24 hours, while retaining its pH at pH 3, which is then heated at 100 °C for 10 minutes to inactivate the enzyme. The resulting mixture is centrifuged at 3000 rpm for 10 minutes to remove the insoluble matters, after which the supernatant is freeze-dried. The resulting peptide has an average molecular weight of 340, in which peptides with molecular weights below 700 occupy 88 % of the peptides.

Examples of the carbohydrates used in the nutrient composition of the present invention are, commercially available lactose, sucrose, glucose, maltose, dextrin, fructose, galactose and so on.

Vitamins used in the nutrient composition of the present invention include, for example, water-soluble vitamins such as vitamin B₁, vitamin B₂, vitamin B₆, vitamin B₁₂, vitamin C, pantothenic acid, niacin, biotin, and folic acid, as well as oil-soluble vitamins such as vitamin A, vitamin D, vitamin E and vitamin K. For such vitamins, any commercial product may be used, and all or several of such vitamins may be appropriately blended as required.

Minerals used in the nutrient composition of the present invention include, for example, commercially available calcium, sodium, phosphorus, chloride, magnesium, iron, zinc, copper, manganese, iodide, and so on. All or several of these minerals may be appropriately blended as required.

The nutrient composition of the present invention may be produced by appropriately blending the individual components. The method by which the nutrient composition of the present invention is produced has been known, and is as described in the following examples.

Next, test examples are described in order to further illustrate the actions and effects of the nutrient composition of the present invention.

### Test Example 1

This test was carried out in order to identify the lipid species suitable for the nutrient composition of the present invention.
1) Sample Preparation
   According to the compositions shown in Table 1, two types of test diets 1 and 2, which differ only in the type of lipid chosen, were prepared by the same method as in Example 1. Because soy bean oil (manufactured by Nippon Oil & Fats Co., Ltd.) composed of long chain triglyceride was blended in test diet 1, test diet 1 contains 4.9 g and 0.7 g per 100 g test diet 1 of the essential fatty acids, linoleic acid and α-linolenic acid, respectively.
   In test diet 2, soy bean phospholipid (manufactured by Lucas Meyer Inc., Germany) with a lipid composition shown in Table 2, where the main component consists of various phospholipids and contains 9.7 % of long chain triglyceride, is blended instead of soy bean oil. Furthermore, as shown in Table 1, the content of linoleic acid and α-linolenic acid in test diet 2 are less than those in test diet 1, which are 2.0 g and 0.3 g per 100 g, respectively.
   Because test diet 3 contains no lipid, it was prepared by the same method as in Example 1, except for the ratio of carbohydrates added, which were increased to adjust the total amount.
2) Test Method
   Test diets 1, 2 and 3 were given to a male patient of age 25 with Type I hyperlipoproteinemia at a dosage of 130 g each, three times a day for 2 separate weeks each. After the completion of each diet ingestion term, blood was collected from the patient to determine the amount of triglyceride in blood by a triglyceride E assay (manufactured by Wako Pure Chemical Industries, Ltd.). The blood was also centrifuged at 3,000 rpm for 15 minutes to obtain plasma, from which the total lipid was extracted by the usual lipid-extraction method using chloroform and methanol, which was then subjected to a silica gel column to fractionate phospholipid. The phospholipid fraction was methylated by using a methanol solution of boron trifluoride, and the fatty acid composition was determined using a gas chromatograph (manufactured by Hewlett Packard Co.) with a capillary column.
3) Test Results
   The test results were as shown in Table 3. The amount of triglyceride in blood after the intake of test diet 1 for 2 weeks was 2,930 mg/dl, showing clinical symptoms of abdominal pain, exanthematic xanthoma, and lipemia of retina. However, after the ingestion term of test diets 2 and 3, which contain almost no long chain triglyceride, the amount of triglyceride in blood showed prominent decreases, being 859 mg/dl and 402 mg/dl, respectively, and the aforesaid clinical symptoms apparently disappeared.

On the other hand, fatty acid analysis of the plasma phospholipid by the usual methods showed that the rsulting compositions of eicosatrienoic acid from test diets 1 and 3 were high, with values of 7.0 % and 7.9 %, respectively, which were much higher than the essential fatty acid index (eicosatrienoic acid/arachidonic acid ratio) reported by T. Holman et al. (American Journal of Clinical Nutrition, Vol. 32, pp. 2390 - 2399, 1979), which were 1.33 and 1.88, respectively, and less than 0.2 for a healthy person. Hence, a state essential fatty acid deficiency was indicated.

The reason for such state may be that because the LPL activity is decreased, when the long chain triglyceride ingested from the diet is digested and absorbed to form triglyceride in chylomicron, the free fatty acids can not be absorbed in the tissue. In contrast, when test diet 2, for which the lipid source is soy bean phospholipid, is ingested, increase in the amount of triglyceride in blood is not observed, and the essential fatty acid index was 0.03, a value in the normal range. It may be suggested that the reason for this is because most of the phospholipid digested and absorbed move directly into the blood where the fatty acid component is hydrolyzed by phospholipase and incorporated to tissues. Therefore, it was proven that in this case, even if the ingested amount of essential fatty acids were enough, because the LPL activity is low, when the ingested form is of long chain fatty acid triglyceride, fatty acids composing the triglyceride of chylomicron are not incorporated in the tissue.

From the above results, it is verified that the reduction of the amount of long chain fatty acid triglyceride intake and the replacement of essential fatty acid supply sources with phospholipids are effective in improving clinical symptoms and essential fatty acid deficiencies.

**Table 1**

| Components | Test diet 1 | Test diet 2 | Test diet 3 |
|---|---|---|---|
| Protein (%) | 17.0 | 17.0 | 17.0 |
| Soy bean oil (%) | 10.0 | - | - |
| Soy bean phospholipid (%) | - | 10.0 | - |
| Carbohydrate (%) | 69.5 | 69.5 | 79.5 |
| Vitamins (%) | 1.0 | 1.0 | 1.0 |
| Minerals (%) | 2.0 | 2.5 | 2.5 |
| Total | 100.0 | 100.0 | 100.0 |
| Linoleic acid (%) | 4.9 | 2.0 | - |
| α-Linolenic acid (%) | 0.7 | 0.3 | - |

**Table 2**

| Components | contents (%) |
|---|---|
| Phosphatidylcholine | 28.5 |
| Phosphatidylethanolamine | 27.2 |
| Phosphatidylinositol | 19.5 |
| Phosphatidic acid | 9.7 |
| Lysophosphatidylcholine | 5.4 |
| Triglyceride | 9.7 |

**Table 3**

| Assay subjects | Test diet 1 | Test diet 2 | Test diet 3 |
|---|---|---|---|
| Blood triglyceride (mg/dl) | 2930 | 859 | 402 |
| Plasma phospholipid fatty acid (%) | | | |
| palmitic acid | 29.3 | 27.2 | 30.4 |
| palmitooleic acid | 3.2 | 0.5 | 3.5 |
| stearic acid | 10.2 | 12.9 | 10.1 |
| oleic acid | 22.6 | 8.2 | 23.6 |
| linoleic acid | 8.4 | 20.2 | 7.1 |
| α-linolenic acid | 0.0 | 0.3 | 0.0 |
| eicosatrienoic acid | 7.0 | 0.3 | 7.9 |
| di-homo-γ-linolenic acid | 3.3 | 5.4 | 2.1 |
| arachidonic acid | 5.3 | 8.9 | 4.2 |
| eicosapentaenoic acid | 0.2 | 0.7 | 0.1 |
| docosahexaenoic acid | 2.0 | 4.4 | 1.2 |
| Essential fatty acid index * | 1.33 | 0.03 | 1.88 |

| | | | |
|---|---|---|---|
| * Essential fatty acid index = eicosatrienoic acid/arachidonic acid (in plasma phospholipid) | | | |

### Test Example 2

This test was carried out, in order to examine the effects of the amount of fat and oil intake on patients with hyperlipoproteinemia
1) Sample Preparation
   As shown in the compositions of Table 4, four types of test diets 1 to 4 which only differ in the amount of soy bean oil (manufactured by Nippon Oils & Fats Co., Ltd.) added, and test diet 5 prepared by substituting the soy bean oil portion of test diet 3 with MCT (manufactured by Taiyo Yushi K.K.) , were prepared by the same method as in Example 1. The fatty acids composition of soy bean oil and MCT are as shown in Table 5. Further, the differences between the amount of fats and oils in each test diet was adjusted with carbohydrates.
2) Test method
   A male patient of age 16 with hyperlipoproteinemia, who shows a significantly low level of LPL activity and a blood triglyceride level of 3,540 mg/dl prior to the ingestion of the test diets, and shows clinical symptoms such as pancreatitis, xanthoma and lipemia of retina, was subjected to a diet of test diets 1 to 4 with various amounts of added fats and oils, at a dosage of 100 g each, three times a day, for 2 separate weeks each. The test diets were powdery, but were liquefied to ease ingestion by appropriately adding water depending on the condition of the patient. The amount of triglyceride in blood was determined by the same method as in the Test Example 1.
3) Test results
   The test results were as shown in Table 6. The amount of triglyceride in the blood, which was 3540 mg/dl prior to the test, was decreased down to 420 mg/dl after the ingestion term of test diet 1, while the clinical symptoms (pancreatitis, xanthoma and lipemia of retina) apparently disappeared. After the ingestion term of test diet 2, the triglyceride amount in blood was at a value slightly larger than that with test diet 1, but no clinical symptoms were observed. After the ingestion term of test diet 3, no clinical symptoms were observed, but blood triglyceride was at a high value of 1010 mg/dl.

According to a report by Murase (Clinical Nutrition, Vol. 78, No. 4, pp. 367-373, 1991), in the case of patients with Type I hyperlipoproteinemia, pancreatitis can be eliminated when blood triglyceride is retained below 1000 mg/dl which can be made effective by controlling the daily fat intake to 30 g or less. However, in Test Example 2, the daily intake of fats and oils is 21.0 g , and the blood triglyceride value is already above 1,000 mg/dl, which suggests that blood triglyceride above this value may possibly cause pancreatitis.

Also, the daily fat intake of healthy males of age 16, the same age as the subject patient, is 76 to 92 g (supervised by the Section of Health Promotion and Nutrition, the Department of Insurance and Medical Treatment, the Ministry of Health and Welfare, The fifth Nutritional Requirement for the Japanese, pp. 8-9, Dai-ichi Shuppan, 1994). However, when the patient ingested test diet 4 which has a far less content of fats and oils, blood triglyceride was increased up to 3,011 mg/dl, and clinical symptoms of pancreatitis and xanthoma were seen.

On the other hand, for test diet 5, the same amount of MCT as that of soy bean oil in test diet 3 was blended. However, the level of blood triglyceride was 510 mg/dl, indicating that blood triglyceride can be controlled to a lower value than with test diet 2, wherein 6.0 g of soy bean oil is ingested daily.

The aforementioned results indicate that in order to control the level of blood triglyceride to below about 1,000 mg/dl and thereby prevent the onset of a clinical symptom, pancreatitis, the daily intake of fats and oils should be controlled below 21.0 g and that the amount of MCT intake hardly increases the level of blood triglyceride.

In order to perform an alimentary therapy for the treatment and prevention of LPL deficiency, hyperchylomicronemia and Type V hyperlipoproteinemia, the intake of lipid from other food should be controlled strictly. The fat intake amount may be adjusted by referring to food component tables (For example, Table of Components of Commercially Available Food by Individual Manufacturers and Products, edited by Yoshiko Kagawa, Jyoshi Eiyo Daigaku Shuppan-bu, 1995) and so on. However, in such cases, inclination in the types of food ingested may occur, causing a deficiency in other nutrients except for lipid. Therefore, the daily diet should be centered on a nutritious food containing all nutrients in good balance; assuming three meals a day, the nutrient composition should contain about 1/3 of the daily amount of fats and oils permitted.

As has been described above, if controlling the amount of fats and oils ingested daily to below 21.0 g is effective, it is essential that the content of fats and oils in the nutrient component is below 7.0 % (by weight) of the total solid content, and the smaller the composition of fats and oils ingested, the more the blood triglyceride is reduced. Therefore, the content of fats and oils in the nutrient composition should preferably be within the range of 0.1 to 2.0 %.

**Table 4**

| Components | Test diet 1 | Test diet 2 | Test diet 3 | Test diet 4 | Test diet 5 |
|---|---|---|---|---|---|
| Protein (%) | 34.0 | 34.0 | 34.0 | 34.0 | 34.0 |
| Soy bean oil (%) | 0.1 | 2.0 | 7.0 | 12.0 | 0.0 |
| MCT (%) | 0.0 | 0.0 | 0.0 | 0.0 | 7.0 |
| Carbohydrates (%) | 61.8 | 59.9 | 54.9 | 49.9 | 54.9 |
| Vitamins (%) | 0.1 | 0.1 | 0.1 | 0.1 | 0.1 |
| Minerals (%) | 4.0 | 4.0 | 4.0 | 4.0 | 4.0 |
| Total (%) | 100.0 | 100.0 | 100.0 | 100.0 | 100.0 |
| Daily intake (g) of fats and oils | 0.3 | 6.0 | 21.0 | 36.0 | 21.0 |

**Table 5**

| Fatty acids | Soy bean oil (%) | MCT (%) |
|---|---|---|
| Caprylic acid | - | 76.4 |
| Capric acid | - | 22.3 |
| Lauric acid | - | 0.1 |
| Palmitic acid | 16.4 | - |
| Stearic acid | 3.9 | - |
| Oleic acid | 7.9 | - |
| Linoleic acid | 59.9 | - |
| α-Linolenic acid | 8.4 | - |
| Others | 3.5 | 1.2 |

**Table 6**

| Test subjects | Prior to test | Test diet 1 | Test diet 2 | Test diet 3 | Test diet 4 | Test diet 5 |
|---|---|---|---|---|---|---|
| Blood triglyceride (mg/dl) | 3540 | 420 | 520 | 1010 | 3011 | 510 |
| Clinical symptoms | pancreatitis xanthoma retinal hyperlipemia | none | none | none | pancreatitis xanthoma | none |

### Test Example 3

This test was carried out, in order to examine the effect of the amount of phospholipid ingested on blood essential fatty acid level of patients with hyperlipoproteinemia
1) Sample Preparation
   Five types of test diets 1 to 5 with compositions shown in Table 7, which differ only in the amount of soy bean phospholipid (manufactured by Lucas Meyer Inc., Germany) were prepared by the same method as in Example 1. Further, the difference in the amount of soy bean phospholipid in each test diet was adjusted with carbohydrates.
2) Test Method
   A patient with Type I hyperlipoproteinemia with LPL deficiency (age 18, male) was subjected to an alimentary therapy wherein the individual test diets 1 to 5 with compositions shown in Table 7, each in the form of a powdered milk, were ingested at a dosage of 120 g, three times a day, for 3 weeks in the same manner as described above. At the end of the each ingestion term, the level of blood triglyceride and the fatty acid composition of the plasma phospholipid were determined by the same method as in the Test Example 1.
3) Test Results
   The test results are as shown in Table 8. The level of blood triglyceride was below 800 mg/dl for each test after the ingestion term of test diets 1 to 4, and no clinical symptom was observed. However at the end of the ingestion term of test diet 5 which contains 20 % of soy bean phospholipid per solid test diet, the level of blood triglyceride was 1,124 mg/dl, which was above 1,000 mg/dl, the level at which pancreatitis may be onset.

It is known that most of the phospholipid absorbed from the small intestine is once hydrolyzed into lysophospholipids and free fatty acids, which are resynthesized to give phospholipids in the epidermal cell of the small intestine to form chylomicron, and that some are synthesized to give triglycerides. Therefore, it is presumed that in the present test, blood triglyceride increased through such a mechanism.

As described above, therefore, for the present disease, it is necessary to limit the amount of long chain triglyceride ingested, as well as to take caution in excess intake of phospholipids. That is, for the prevention and treatment of the disease, it is effective to ingest an appropriate amount of highly pure phospholipid selected from various commercial phospholipid products. Therefore, it is preferable that the phospholipid used has a purity of 80 % or more with as little acetone-soluble neutral lipid as possible.

Additionally, the essential fatty acid index, or the ratio of eicosatrienoic acid/arachidonic acid in plasma phospholipid at the end of the ingestion term of test diet 1 was 1.80, a value much higher than that of healthy subjects, reported by Holman et al., which is below 0.2, as described before. Hence, a state of essential fatty acid deficiency was indicated. When test diet 2 containing 5 % per solid of soy bean phospholipid was ingested, the essential fatty acid index was 0.09, showing improvement in essential fatty acid deficiency. Since the more the amount of soy bean phospholipid blended, the better improved the condition of essential fatty acid deficiency, it was proven that in order to prevent the blood triglyceride from increasing to a value above 1,000mg/dl, the amount of soy bean phospholipid blended should preferably be within the range of 7.0 to 15.0 % of the total solid content.

The aforementioned results verify that phospholipid of at least 5.0%, preferably 7.0 % to 15.0 % in the total solid content of the nutrient composition is effective to improve the state of essential fatty acid deficiency in LPL-deficient patients with Type I hyperlipoproteinemia.

**Table 7**

| Components | Test diet 1 | Test diet 2 | Test diet 3 | Test diet 4 | Test diet 5 |
|---|---|---|---|---|---|
| Protein (%) | 18.0 | 18.0 | 18.0 | 18.0 | 18.0 |
| Soy bean phospholipid (%) | 1.0 | 5.0 | 7.0 | 15.0 | 20.0 |
| Carbohydrates (%) | 77.7 | 73.7 | 71.7 | 63.7 | 58.7 |
| Vitamins (%) | 0.3 | 0.3 | 0.3 | 0.3 | 0.3 |
| Minerals (%) | 3.0 | 3.0 | 3.0 | 3.0 | 3.0 |
| Total (%) | 100.0 | 100.0 | 100.0 | 100.0 | 100.0 |

**Table 8**

| Assay subjects | Test diet 1 | Test diet 2 | Test diet 3 | Test diet 4 | Test diet 5 |
|---|---|---|---|---|---|
| Blood triglyceride (mg/dl) | 430 | 450 | 512 | 720 | 1124 |
| Plasma phospholipid fatty acid (%) palmitic acid | 30.3 | 28.2 | 27.7 | 26.0 | 25.4 |
| palmitooleic acid | 6.2 | 2.1 | 1.7 | 0.9 | 0.3 |
| stearic acid | 10.2 | 12.9 | 10.1 | 9.5 | 10.1 |
| oleic acid | 22.6 | 18.6 | 15.1 | 13.5 | 8.5 |
| linolenic acid | 7.9 | 14.6 | 19.4 | 22.1 | 26.4 |
| α-linolenic acid | 0.1 | 0.3 | 0.4 | 0.5 | 0.8 |
| eicosatrienoic acid | 7.2 | 0.5 | 0.4 | 0.2 | 0.1 |
| di-home-γ-linolenic acid | 2.1 | 3.9 | 5.1 | 6.5 | 8.1 |
| arachidonic acid | 4.0 | 5.7 | 6.5 | 8.0 | 8.5 |
| eicosapentaenoic acid | 0.2 | 0.5 | 0.8 | 0.9 | 1.0 |
| docosahexaenoic acid | 1.9 | 2.4 | 3.5 | 3.9 | 4.4 |
| Essential fatty acid index * | 1.80 | 0.09 | 0.06 | 0.03 | 0.01 |

| | | | | | |
|---|---|---|---|---|---|
| (*) the same as in Table 3. | | | | | |

Next, Examples are described to illustrate the nutrient composition of the present invention more thoroughly and specifically. However, the present invention is not limited to the following examples.

### Example 1

7.0 kg of commercially available casein powder (manufactured by New Zealand Dairy Board) and 9.7 kg of commercially available whey protein (manufactured by Milei GmbH, Germany) were dissolved in 150 liters of warm water to obtain a solution, to which were added 56.0 kg of commercially available lactose (manufactured by Milei GmbH, Germany), 10.3 kg of dextrin (manufactured by Matsutani Chemical Industry Co.), 8.0 kg of commercially available soy bean phospholipid (manufactured by Lucas Meyer Inc., Germany; phospholipid content 90.3 %), 5.0 kg of commercially available MCT (manufactured by Taiyo Yushi K.K.), 2.0 kg of vitamins and 2.0 kg of minerals, which was then uniformly mixed to homogenization, sterilized under heating at 120 °C for 3 seconds and spray-dried by a general method, to obtain 100kg of powdered milk for the prevention or treatment of hyperlipoproteinemia.

### Example 2

10.4 kg of commercially available casein powder (manufactured by New Zealand Dairy Board) and 9.0 kg of commercially available whey protein (manufactured by Milei GmbH, Germany) were dissolved in 60 liters of warm water. To the resulting solution, 65.9 kg of commercially available dextrin (manufactured by Matsutani Chemical Industry Co.), 7.9 kg of commercially available soy bean phospholipid (manufactured by Lucas Meyer Inc., Germany; phospholipid content 90.3 %), 2.0 kg of commercially available MCT (manufactured by Taiyo Yushi K.K.), 2.0 kg of vitamins and 2.8 kg of minerals were added. The solution was then mixed uniformly and homogenized, adjusted to a total volume of 100 liters, and sterilized under heating at 150 °C for 2 seconds, to obtain 100 kg of liquid enteral alimentation for the prevention or treatment of hyperlipoproteinemia.

### Example 3

A powdered milk of the following composition for the prevention or treatment of hyperlipoproteinemia in infants was prepared according to general methods. Commercially available products were used for all components except casein powder (manufactured by New Zealand Dairy Board), soy bean phospholipid (manufactured by Lucas Meyer Inc., Germany; phospholipid content 90.3 %), MCT (manufactured by Taiyo Yushi K.K.), lactose (manufactured by Milei GmbH, Germany) and dextrin (manufactured by Matsutani Chemical Industry Co.).

| | (g/100 g) |
|---|---|
| Casein powder | 6.2 |
| Whey protein | 8.2 |
| Soy bean phospholipic | 6.0 |
| MCT | 5.0 |
| Lactose | 15.4 |
| Dextrin | 54.0 |
| Vitamins | 2.0 |
| Minerals | 3.2 |

### Example 4

17.0 kg of commercially available soy bean protein (manufactured by Fuji Oil Co., Ltd.) was dissolved in 150 liters of warm water at 60 °C. To the mixture were added 6.0 kg of egg yolk phospholipid (manufactured by Kewpie, Co., Ltd.; phospholipid content 95 %), 70.8 kg of dextrin (manufactured by Matsutani Chemical Industry Co.), 0.5 kg of raffinose (manufactured by Nippon Beet Sugar Manufacturing Co., Ltd.), 0.5 kg of lactulose (manufactured by Morinaga Milk Industry Co., Ltd.), 2.0 kg of commercially available vitamins and 3.2 kg of commercially available minerals. The resulting mixture was uniformly mixed and homogenized, sterilized under heating at 120 °C for 3 seconds, and spray-dried by the usual methods, to obtain about 100 kg of powdered milk for the prevention or treatment of hyperlipoproteinemia, from which arachidonic acid and docosahexanoic acid in egg yolk lecithin can be ingested.

### Industrial Applicability

The present invention provides a nutrient composition for the prevention or treatment of hyperlipoproteinemia, which causes no essential fatty acid deficiency, does not increase the level of undesirable fat in blood, and can supply enough energy. Because the nutrient composition of the present invention can be used for the prevention or treatment of hyperlipoproteinemia in a wide range of ages, from infants to the elderly, this nutrient composition can be applied to powdered milk for specific uses, various fluid diets, powdered milk preparations, and so on. Furthermore, this nutrient composition has a low content of fats and oils, yet contains the required amount of essential fatty acids; thus, it can be used to supplement essential fatty acids to patients with lipid absorption disorders.

## Claims

1. A nutrient composition for the prevention or treatment of hyperlipoproteinemia, wherein the nutrient composition contains one or two or more nitrogen sources selected from the group consisting of protein, protein hydrolysates, peptides and amino acid, lipids, carbohydrate, vitamins and minerals, wherein the lipid comprises one of the following a) or b) :
a) phospholipids of at least 5.0 % (by weight) of the total solid content;
b) phospholipids of at least 5.0 % (by weight) of the total solid content, and fats and oils of less than 7.0 % (by weight) of the total solid content (provided that 0 % (by weight) is excluded).

2. A nutrient composition according to claim 1, wherein the content of fats and oils occupies 0.1 to 2.0 % (by weight) of the total solid content.

3. A nutrient composition according to claims 1 or 2, wherein the purity of phospholipid is at least 80 % (by weight).
